# EUROPÄISCHE PATENTANMELDUNG

(11) **EP 3 575 336 A1**
(43) Veröffentlichungstag der Anmeldung: **04.12.2019**
(21) Anmeldenummer: 18175075.3
(22) Anmeldetag: 30.05.2018
(51) Int. Cl.: C08G 18/32, C08G 65/26

(54) **ZUSAMMENSETZUNG ENTHALTEND BISPHENOL F**

(71) Anmelder: Hexion GmbH, 58642 Iserlohn-Letmathe (DE)
(72) Erfinder: Schröter, Stephan, 45259 Essen (DE); Kukkala, Pravin, Louisville, KY 40210 (US); Viswanathan, Ganapathy, Louisville, KY 40223 (US); Maiorana, Anthony, Louisville, KY 40204 (US); Kerkaidou, Athina, 58644 Iserlohn (DE)

(57) **Zusammenfassung**

Die Erfindung bezieht sich auf eine Zusammensetzung, die eine binäre Mischung aus ethoxyliertem Bisphenol F und propoxyliertem Bisphenol F enthält.

Um aromatische Polyole zur Herstellung von Polymeren auf Polyurethan- und Polyisocyanuratbasis zur Verfügung zu stellen, die einerseits eine gute prozesstechnische Handhabung und eine gute Vermischbarkeit mit der Isocyanatkomponente gewährleisten und andererseits dem Endprodukt eine gute Flammfestigkeit verleihen, wird eine Zusammensetzung vorgeschlagen, die eine binäre Mischung aus ethoxyliertem Bisphenol F und propoxyliertem Bisphenol F in einem Gewichtsverhältnis von 20:80 bis 80:20 enthält.

## Beschreibung

Bei der Herstellung von Polyurethanen und Polyisocyanuraten werden Isocyanate unter Verwendung von Polyolen durch eine Polyadditionsreaktion vernetzt. Die Isocyanate weisen mindestens zwei -NCO-Gruppen und die Polyole mindestens zwei reaktionsfähige -OH-Gruppen (mehrwertige Alkohole) auf. Es entstehen Polymere die je nach ihrem chemischen oder morphologischen Aufbau thermoplastische, elastische oder duroplastische Eigenschaften aufweisen können. Demzufolge besitzen Polyurethane und Polyisocyanurate ein sehr breites Anwendungsgebiet, wie z.B. für Schäume, Beschichtungen, Klebstoffe, Elastomere, Isolationen und Kompositwerkstoffe. Insbesondere bei der Suche nach energie- und ressourceneffizienten Werkstoffen, kommt den Polyurethanen und Polyisocyanuraten aufgrund einfach zu realisierenden Leichtbaukonstruktionen eine besonders große Bedeutung zu.

Die Herstellung von Polyurethanen und Polyisocyanuraten erfolgt durch Mischen der Polyole mit Isocyanten, wobei nach kurzer Zeit das System zu gelieren beginnt. Es ist ersichtlich, dass die Komponenten hinsichtlich ihrer Viskositäten aufeinander abgestimmt sein müssen, damit ein Vermischungsgrad vorliegt, der zu homogenen Produkten mit gewünschten Eigenschaften führt. Die Eigenschaften des Endproduktes werden im Wesentlichen durch die Kettenlänge und Verzweigungsgrad der Polyolkomponente bestimmt, so dass häufig auch Kombinationen aus verschiedenen Polyolen eingesetzt werden, z.B. Polyetherpolyole und Polyesterpolyole, um Verarbeitung und Eigenschaften zu optimieren.

Bei der Herstellung typischer Polyurethan-Hartschaumstoffe führt die stark exotherme Reaktion zwischen Polyolen und Isocyanaten zu innerer Verkrustung. Dieses Phänomen beeinträchtigt die physikalischen Eigenschaften des Schaums nachteilig und erhöht das Potential, Probleme hinsichtlich der Entflammbarkeit zu verursachen. Darüber hinaus erhöht die Verwendung von organischen Treibmitteln auf Kohlenwasserstoffbasis die Entflammbarkeit der fertigen Schaumstoffe. Folglich werden flammhemmende Additive in die Formulierung gegeben, wobei dies im Allgemeinen halogenierte Verbindungen sind. Viele solcher Flammschutzmittel stellen jedoch eine Gefahr für die Umwelt dar. Daher ist es wünschenswert, Polyole zu verwenden, die von Natur aus schwer entflammbar sind, die die Wärme- und Feuerbeständigkeit der Polyurethan (PUR) - und Polyisocyanurat (PIR) - Schäume verbessern und möglicherweise die Mengen dieser umweltfreundlichen und teuren Flammschutzadditive in der Formulierung minimieren,

Alkoxylierte Bisphenole sind für die Herstellung von Polyurethanen bekannt. So beschreibt die EP 0 763 067 B1 die Verwendung von alkoxylierten Bisphenolen für die Herstellung von Schmelzklebern und die EP 2 743 285 A1 für beschichtete Leitungselemente. Weiterhin kann der EP 1 851 261 B1 entnommen werden, dass eine Komponente einer zweikomponentigen Polyurethanzusammensetzungen für Strukturklebstoffe ein ethoxyliertes oder propoxyliertes aromatisches Diol in Kombination mit aliphatischen Triolen sein kann.

Es hat sich aber herausgestellt, dass aromatische Polyole vom Dioltyp auf der Basis von Bisphenol A für die Herstellung von Polyurethanen nur unzureichend geeignet sind, da sie feste Substanzen sind, was verarbeitungstechnisch sehr nachteilig ist und außerdem noch eine schlechte thermische Beständigkeit besitzen.

Es hat sich auch gezeigt, dass die Verwendung von sowohl reinem ethoxylierten Bisphenol F als auch reinem propoxylierten Bisphenol F als aromatische Diole zur Herstellung von Polyurethanen eine ernste Herausforderung darstellt. Grund hierfür ist, dass es sich jeweils um pastöse Substanzen handelt, die bei 20-30°C nicht pumpfähig sind und somit nicht die gewünschte prozesstechnische Verarbeitungsfähigkeit in der Polyurethanherstellung besitzen. Ein Aufschmelzen des ethoxylierten Bisphenol F bzw. des propoxylierten Bisphenol F setzt ein Energieeintrag in das System voraus, was auch nicht erwünscht ist. Weiterhin zeigten Versuche reines ethoxyliertes Bisphenol F als auch reines propoxyliertes Bisphenol F in Lösung zu bringen, dass sie nicht in geeigneten Lösungsmittel aufgelöst werden konnten, da sie leicht zur Kristallisation neigten.

Es ist daher Aufgabe der hier vorliegenden Erfindung aromatische Polyole zur Herstellung von Polymeren auf Polyurethan- und Polyisocyanuratbasis zur Verfügung zu stellen, die einerseits eine gute prozesstechnische Handhabung bei ca. 20°C und eine gute Vermischbarkeit mit der Isocyanatkomponente gewährleisten und andererseits dem Endprodukt eine gute Flammfestigkeit verleihen, wobei auf den Einsatz von halogenierten Verbindungen verzichtet werden kann.

Gelöst wird diese Aufgabe erfindungsgemäß durch eine Zusammensetzung, die eine binäre Mischung aus ethoxyliertem Bisphenol F und propoxyliertem Bisphenol F in einem Gewichtsverhältnis von 20:80 bis 80:20 enthält.

Hierbei wird unter dem Begriff binäre Mischung eine rein physikalische Mischung zweier separater Komponenten, nämlich ethoxyliertem Bisphenol F und propoxyliertem Bisphenol F verstanden. Alkoxyliertes Bisphenol F, was durch Umsetzung eines Ethoxylierungsmittel und Propoxylierungsmittel mit Bisphenol F entstanden ist und damit ein Co-Kondensat darstellt, soll somit nicht unter den Begriff "binäre Mischung" fallen.

Überraschenderweise wurde festgestellt, dass sich binäre Mischungen von ethoxyliertem Bisphenol F und propoxyliertem Bisphenol F in einem Gewichtsverhältnis von 20: 80 bis 80:20 in verschiedenen Lösungsmitteln, die sich auch mit Isocyanaten vermischen lassen, hervorragend lösen. Das war nicht zu erwarten, da - wie bereits erwähnt - sowohl ethoxyliertes Bisphenol F als auch propoxyliertes Bisphenol F leicht zur Kristallisation neigen.

Das zur Herstellung von ethoxyliertem bzw. propoxyliertem Bisphenol F verwendete Bisphenol F ist aus dem Stand der Technik bekannt. So wird es durch Umsetzung von Phenol mit Formaldehyd im Sauren hergestellt. Es entsteht ein Isomerengemisch aus o-o', o-p und p-p' Bisphenol F, was je nach Reaktionsbedingungen und Herstellverfahren in der Zusammensetzung variieren kann. Die Hydroxyfunktionalität des hergestellten Bisphenol F beträgt mindestens 2.

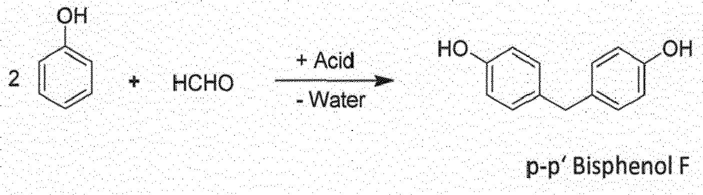

Durch Umsetzung des Bisphenol F mit Ethoxylierungsmitteln, wie Ethylenoxid oder Ethylencarbonat, entsteht das ethoxylierte Bisphenol F. Entsprechend erfolgt die Umsetzung des Bisphenol F mit Propoxylierungsmitteln, wie Propylenoxid oder Propylencarbonat, zum propoxylierten Bisphenol F. Da die Umsetzung mit Ethylenoxid bzw. Propylenoxid, die im gasförmigen Zustand vorliegen, in einem Druckreaktor erfolgen muss, was prozesstechnisch aufwendiger ist, wird bevorzugt als Ethoxylierungsmittel Ethylencarbonat bzw. als Propoxylierungsmittel Propylencarbonat verwendet.

Am Beispiel des p-p' Bisphenol F soll die Reaktion verdeutlicht werden:

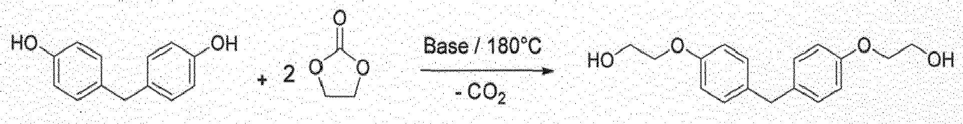

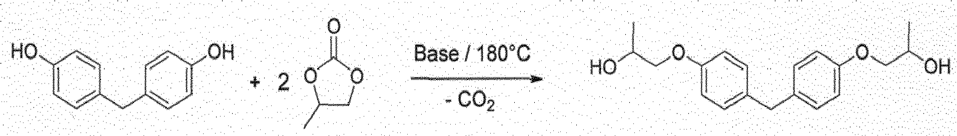

Aufgrund der verschiedenen Isomere des Bisphenol F entstehen auch entsprechende Isomere des ethoxylierten bzw. proproxylierten Produktes:

| Isomere des ethoxylierten Bisphenol F | Isomere des proproxylierten Bisphenol F |
|---|---|
| 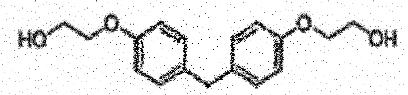 | 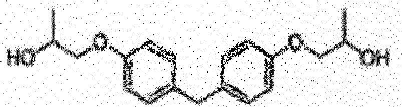 |
| 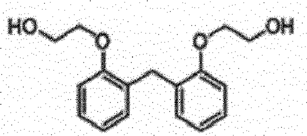 | 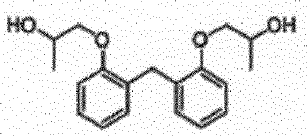 |
| 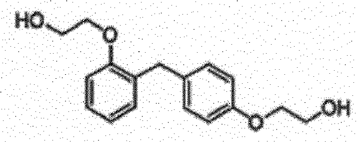 | 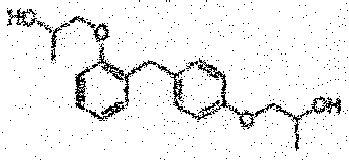 |

Generell wird die Ethoxylierungs- bzw. Propoxylierungsreaktion im alkalischen Medium bei Temperaturen zwischen 120 und 200 °C durchgeführt. In der Regel wird das Bisphenol F vorgelegt, aufgeschmolzen und es erfolgt die Zugabe eines alkalischen Mediums in Form von z.B. Kaliumcarbonat, Natriumcarbonat, Kaliumhydroxid, Natriumhydroxid, Calciumhydroxid, Calciumoxid, Aminen oder Triphenylphosphin bei Temperaturen bis 180 °C. Anschließend wird Ethylencarbonat bzw. Propylencarbonat zugegeben und je nach technischer Möglichkeit das entstehende Kohlendioxid abgeführt. Das entstandene Produkt muss nicht zwangsläufig abdestilliert werden und kann nach Abkühlung gegebenenfalls mit einer Säure neutralisiert werden. Ein neutrales Produkt wird bevorzugt bei der Anwendung als Polyol bei der Polyurethanherstellung. Bevorzugt ist eine Neutralisation z.B. mit einer kompatiblen organischen (wie z.B. Benzoesäure, Phthalsäure, Milchsäure, Anthranilsäure oder Salicylsäure und/oder anorganischen Säure (wie z.B. Salzsäure, Schwefelsäure, Phosphorsäure oder Salpetersäure).

Bevorzugt ist ein Verfahren zur Herstellung der erfindungsgemäßen Zusammensetzung folgende Schritte enthaltend:
I)
   a) Zugabe von Bisphenol F in einen Reaktor und bei Temperaturen zwischen 120 °C und 160 °C aufschmelzen
   b) Zugabe eines alkalischen Mediums und temperieren bis auf 180 °C
   c) Zugabe von Ethylencarbonat und Abführung des Kohlendioxides
   d) gegebenenfalls Zugabe einer Säure zur Neutralisation
II)
   e) Zugabe von Bisphenol F in einen Reaktor und bei Temperaturen zwischen 120 °C und 160 °C aufschmelzen
   f) Zugabe eines alkalischen Mediums und temperieren bis auf 180 °C
   g) Zugabe von Propylencarbonat und Abführung des Kohlendioxides
   h) gegebenenfalls Zugabe einer Säure zur Neutralisation
III) Zusammenführen der unter (I) und (II) hergestellten Komponenten, bevorzugt in einem Lösungsmittel, zur Herstellung der binären Mischung.

Das Molverhältnis von Bisphenol F : Ethoxylierungsmittel bzw. Propoxylierungsmittel kann z.B. 1:4 bis 1:2 betragen. Je mehr Ethoxylierungsmittel bzw. Propoxylierungsmittel bei der Herstellung verwendet wurde (z.B. 1:4), umso besser war die Löslichkeit in verschiedenen Lösungsmitteln und somit die Vermischbarkeit mit dem Isocyanat. Wenn das Verhältnis Bisphenol F : Ethoxylierungsmittel und/oder das Verhältnis Bisphenol F : Propoxylierungsmittel 1:2,0 bis 1:2,3, bevorzugt 1:2 gewählt wurde, konnte durch den höheren Aromatenanteil im Polyurethanwerkstoff die Flammbeständigkeit weiter erhöht werden, wobei immer noch exzellente Löslichkeiten in verschiedenen Lösungsmittel zu verzeichnen waren.

Generell ist es auch möglich die Herstellung des Bisphenol F durch Reaktion von Phenol mit Formaldehyd in einem Reaktor durchzuführen und direkt anschließend vorzugsweise in demselben Reaktionsgefäß die Umsetzung mit dem Ethoxylierungsmittel oder Propoxylierungsmittel vorzunehmen. Das hat den Vorteil, dass eine Lagerung des Bisphenol F nicht erforderlich wäre und bedarfsgerecht sofort weiterverwendet werden kann.

Nach der erforderlichen separaten Herstellung des ethoxylierten Bisphenol F und propoxylierten Bisphenol F werden diese beiden Substanzen zu einer binären Mischung - vorzugsweise unter Rühren - in einem Gewichtsverhältnis von 20: 80 bis 80:20, bevorzugt 30:70 bis 70:30, wiederum bevorzugt 50:50, bevorzugt in einem Lösungsmittel zusammengeführt. Generell ist es aber auch möglich, das ethoxylierte Bisphenol F und das propoxylierte Bisphenol F zu erwärmen und in dem angegebenen Gewichtsverhältnis miteinander innig zu vermischen und dann gegebenenfalls anschließend das Lösungsmittel zuzugeben.

Die binäre Mischung zeigt überraschenderweise eine sehr gute Löslichkeit in Lösungsmitteln, die insbesondere mit der Herstellung von Polyurethanen kompatibel sind. So löst sich die binäre Mischung sehr gut in Organophosphaten wie Triethylphosphat und Diphenylkresylphosphat, 1,4-Butandiol, Polyetherpolyolen wie ethoxyliertem Zucker, aromatischen Polyesterpolyolen, modifizierten oder unmodifizierten phenolischen Resolen (z.B. Phenol und Kresol basierte Resole) allein oder in Mischungen hiervon. Die Resole können auch in organischem Lösungsmittel gelöst vorliegen.

Die Kristallisationsneigung der erfindungsgemäßen Zusammensetzung war überraschenderweise im Vergleich zu rein ethoxyliertem Bisphenol F oder propoxylierten Bisphenol F enthaltenden Lösungen sehr gering bzw. nicht vorhanden. Dabei wurde die binäre Mischung bevorzugt im Gewichtsverhältnis zum Lösungsmittel 80:20 bis 20:80, bevorzugt 60:40 bis 40:60, wiederum bevorzugt 50:50 zugegeben. Insbesondere bei einem Verhältnis binäre Mischung zu Lösungsmittel von 50:50 zeigte die erfindungsgemäße Zusammensetzung über mehrere Wochen eine sehr gute Lagerstabilität in verschiedenen Lösungsmitteln.

Als besonders bevorzugt hat sich als Lösungsmittel alkoxyliertes Resorcin bewährt. Unter dem Begriff "alkoxyliertes Resorcin" fallen Substanzen, bei denen das Resorcin mit mindestens einem Alkoxylierungsmittel, so z.B. einem Ethoxylierungsmittel (Ethylenoxid, Ethylencarbonat) und/oder Propoxylierunsgmittel (Propylenoxid, Propylencarbonat) umgesetzt wurde. Das molare Verhältnis von Resorcin (Resorcinol) zu Alkoxylierunsgmittel beträgt bevorzugt 1: 2 bis 1: 2,5.

Unter alkoxyliertem Resorcin ist z.B. folgende Struktur zu fassen: 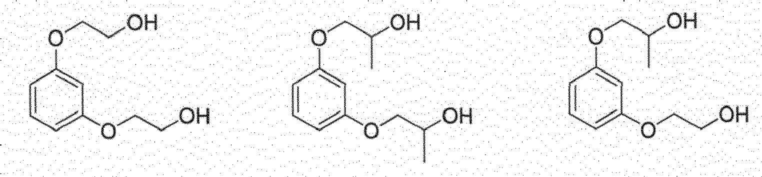 wobei diese Produkte hauptsächlich bei der Reaktion von Resorcin mit Ethylencarbonat und Propylencarbonat entstehen. Es können aber noch weitere Produkte, insbesondere bei der Reaktion mit Propylencarbonat gebildet werden, da in Propylencarbonat zwei unterschiedliche C-Atome angegriffen werden. Ebenso werden verschiedene Stereoisomere bei der Reaktion mit Propylencarbonat erhalten.

Besonders bevorzugt ist, wenn als Alkoxylierungsmittel eine Kombination aus einem Ethoxylierungsmittel und einem Propoxylierungsmittel verwendet wird. Ein solch alkoxyliertes Produkt wirkte als Lösungsmittel der binären Mischung wirksamer als z.B. reines propoxyliertes Resorcin.

Die Herstellung des alkoxylierten Resorcins kann in der Art erfolgen, dass das Resorcin aufgeschmolzen wird und im alkalischen Medium in Form von z.B. Kaliumcarbonat, Natriumcarbonat, Kaliumhydroxid, Natriumhydroxid, Calciumhydroxid, Calciumoxid, Aminen oder Triphenylphosphin bei erhöhten Temperaturen mit einem ersten Alkoxylierungsmittel (z.B. Propylencarbonat) versetzt wird und die Reaktion unter Abführung von Kohlendioxid stattfindet. Daran anschließen kann sich gegebenenfalls die Zugabe eines weiteren Alkoxylierungsmittel (z.B. Ethylencarbonat) bei erhöhter Temperatur, wobei das Kohlendioxid wiederum abgeleitet wird. Nach entsprechender Nachreaktion kann das Produkt gegebenenfalls abdestilliert und mit einer Säure (wie z.B. Benzoesäure, Phthalsäure, Milchsäure, Anthranilsäure, Salicylsäure Salzsäure, Schwefelsäure, Phosphorsäure und/oder Salpetersäure) neutralisiert werden.

Die Verwendung von alkoxylierten Resorcin ist insbesondere bei der Herstellung von Polyurethanen von Vorteil, da zusätzliche difunktionelle Gruppen vorhanden sind, die mit Isocyanaten reagieren können. Gleichzeitig wird die Viskosität der gesamten Polyolmischung so beeinflusst, dass sie eine gute Lagerstabilität zur Folge hat. Weiterhin erhöht die Verwendung von alkoxylierten Resorcin den aromatischen Anteil der Polyolkomponente, so dass die Flammbeständigkeit und die Kompatibilität z.B. zum MDI oder den Treibmitteln weiter erhöht werden konnte.

Verfahrenstechnisch günstig ist es, wenn bei der Herstellung des ethoxylierten Bisphenol F oder des propoxylierten Bisphenol F das Resorcin gleichzeitig oder im Anschluss unter Ausnutzung desselben Reaktionsgefäßes das Resorcin ethoxyliert und/oder propoxyliert wird.

Das Verhältnis der binären Mischung zum alkoxylierten Resorcin liegt in der erfindungsgemäßen Zusammensetzung im Gewichtsverhältnis 80:20 bis 20:80, bevorzugt 60:40 bis 40:60, wiederum bevorzugt 50:50. So könnte z.B.in einer Ausführungsform das Verhältnis EBF:PBF:ARC gleich 25:25:50 sein.

Die in Lösung gebrachte binäre Mischung neigt überrascherweise nicht zur Kristallisation und bildet eine lagerstabile Zusammensetzung, die als Polyolkomponente bei Bedarf mit Di- oder Poly-Isocyanaten zur Herstellung von Polyurethanen oder Polyisocyanurat basierten Polymeren verwendet werden kann.

Generell ist es auch möglich, dass die erfindungsgemäße Zusammensetzung als eine Polyolkomponente verwendet wird, d.h. dass noch weitere Polyolkomponenten zur Herstellung von Polyurethanen bzw. Polyisocyanuraten eingesetzt werden, wie z.B. Polyesterpolyolen. Polyesterpolyole umfassen Reaktionsprodukte von Polyolen, üblicherweise Diolen, mit Polycarbonsäuren oder ihren Anhydriden, üblicherweise Dicarbonsäuren oder Dicarbonsäureanhydriden. Die Polycarbonsäuren oder -anhydride können aliphatisch, cycloaliphatisch, aromatisch und / oder heterocyclisch sein. Mannich-basische Polyole, die aus Mannichbasen synthetisiert werden, können auch als Teil der isocyanatreaktiven Verbindung verwendet werden.

Als Isocyanatkomponente kommen bevorzugt m-Phenylendiisocyanat, Toluol-2,4-diisocyanat, Toluol-2,6-diisocyanat, Hexamethylen-1,6-diisocyanat, Tetramethylen-1,4-diisocyanat, Cyclohexan-1,4-diisocyanat, Hexahydrotoluoldiisocyanat, Naphthylen-1,5-diisocyanat, Methoxyphenyl-2,4-diisocyanat, Diphenylmethan-4,4'-diisocyanat, 4,4'-Biphenylendiisocyanat, 3,3'- Dimethoxy-4,4'-biphenyldiisocyanat, 3,3'-Dimethyl-4,4'-biphenyldiisocyanat, 3,3'-Dimethyldiphenylmethan-4,4'-diisocyanat, 4,4', 4 "-Triphenylmethan Triisocyanat, ein Polymethylenpolyphenylisocyanat, polymeres Diphenylmethandiisocyanat (PMDI), Isophorondiisocyanat, Toluol-2,4,6-triisocyanat und 4,4'-Dimethyldiphenylmethan-2,2', 5,5'-tetraisocyanat zum Einsatz. In verschiedenen Ausführungsformen ist das Polyisocyanat Diphenylmethan-4,4'-diisocyanat, Diphenylmethan-2,4-diisocyanat, Hexamethylen-1,6-diisocyanat, Isophorondiisocyanat, Toluol-2,4-diisocyanat, Toluoi-2,6-diisocyanat oder Mischungen davon. Diphenylmethan-4,4'-diisocyanat, Diphenylmethan-2,4-diisocyanat und Mischungen davon werden im Allgemeinen als MDI bezeichnet.Toluol-2,4-diisocyanat, Toluol-2,6-diisocyanat und Mischungen davon werden allgemein als TDI bezeichnet.

Jedes der vorstehenden Polyisocyanat kann so modifiziert werden, dass Urethan-, Harnstoff-, Biuret-, Carbodiimid-, Allophonat-, Uretonimin-, isocyanurat-, Amid- oder ähnliche Bindungen eingeschlossen sind. Beispiele für modifizierte isocyanate dieser Art umfassen verschiedene Urethangruppen- und/oder Harnstoffgruppen enthaltende Präpolymere und sogenannte "Flüssig-MDI" -Produkte und dergleichen. Es ist auch möglich, dass das Polyisocyanat ein blockiertes Isocyanat ist.

In Abhängigkeit von dem speziellen Typ des hergestellten Polymers und den notwendigen Eigenschaften des Polymers kann eine große Vielzahl von zusätzlichen Materialien während der Reaktion der Polyisocyanatverbindung mit der erfindungsgemäßen Zusammensetzung vorhanden sein. Diese Materialien umfassen, ohne darauf beschränkt zu sein, Tenside, Treibmittel, Zellöffner, Füllstoffe, Pigmente und/oder Färbemittel, Trocknungsmittel, Verstärkungsmittel, Biozide, Konservierungsmittel, Antioxidantien, Verdünnungsmittel, Flammschutzmittel und dergleichen. Wenn ein Flammschutzmittel enthalten ist, kann das Flammschutzmittel ein phosphorhaltiges Flammschutzmittel sein. Beispiele für phosphorhaltige Flammschutzmittel umfassen, sind aber nicht beschränkt auf, Triethylphosphat (TEP), Triphenylphosphat (TPP), Trischlorisopropylphosphat (TCPP), Dimethylpropanphosphat, Resorcinolbis-(diphenylphosphat) (RDP), Bisphenol-A-Diphenylphosphat (BADP) und Trikresylphosphat (TCP), Dimethylmethylphosphonat (DMMP), Diphenylkresylphosphat und Aluminiumdiethylphosphinat. Beispiele für Verdünnungsmittel umfassen Polyglycole wie Ethylenglycol, Glycerol oder Diethylenglycol, veretherte Polyglycole wie Monomethylether von Ethylenglycol oder Dimethylether von Ethylenglycol und zweibasische Ester von Säuren wie Diethyladipat, Dimethyladipat, Diethylsuccinat oder Dimethylsuccinat. Mischungen dieser Verdünnungsmittel können ebenfalls verwendet werden.

Die relativen Mengen von Polyisocyanat und erfindungsgemäßer Zusammensetzung werden ausgewählt, um ein Polymer zu erzeugen. Das Verhältnis dieser Komponenten wird im Allgemeinen als der "isocyanatindex" bezeichnet, was das 100-fache des Verhältnisses von Isocyanatgruppen zu isocyanatreaktiven Gruppen bedeutet, die durch die erfindungsgemäße Zusammensetzung bereitgestellt werden. Der Isocyanatindex beträgt im Allgemeinen mindestens 50 und kann bis zu 1000 oder mehr betragen. Starre Polymere wie Strukturpolyurethane und Hartschaumstoffe werden typischerweise unter Verwendung eines Isocyanat-Index von 90 bis 200 hergestellt. Wenn flexible oder halbflexible Polymere hergestellt werden, beträgt der isocyanatindex im allgemeinen 70 bis 125. Polymere, die Isocyanuratgruppen enthalten, werden oft hergestellt mit Isocyanat-Indizes von mindestens 150 bis zu 600 oder mehr.

Um das Polymer zu bilden, werden die Polyisocyanatverbindung und die erfindungsgemäße Zusammensetzung gemischt und zur Reaktion gebracht.

Gegebenenfalls kann das Polyisocyanat und die erfindungsgemäße Zusammensetzung in verschiedenen Ausführungsformen auch einen Katalysator enthalten. Beispiele für Katalysatoren schließen tertiäre Amine, wie Dimethylbenzylamin, 1,8-Diaza (5,4,0) undecan-7, Pentamethyldiethylentriamin, Dimethylcyclohexylamin und Triethylendiamin ein, sind aber nicht darauf beschränkt. Kaliumsalze, wie Kaliumacetat und Kaliumoctoat, können ebenfalls als Katalysatoren verwendet werden.

Die erfindungsgemäße Zusammensetzung kann zur Herstellung von Polyurethanen insbesondere in Form von Präpolymeren, Schäumen (fest, flexibel), Beschichtungen, Lacken, Elastomeren, Klebstoffen, Dichtmitteln und/oder Kompositwerkstoffen verwendet werden.

Anhand eines Ausführungsbeispiels soll die Erfindung näher erläutert werden:
a) Herstellung von ethoxylierten Bisphenol F (EBF)
   1. 694,20 kg Bisphenol F werden als Feststoff in einen Reaktor gegeben und bei Temperaturen zwischen 120 °C - 160 °C aufgeschmolzen.
   2. 1,74 kg Kaliumcarbonat werden anschließend unter Rühren bei 130 °C zugegeben und die Reaktionsmischung weiter auf 175 °C - 180 °C aufgeheizt.
   3. Danach werden 611,60 kg Ethylencarbonat in 5 h unter Rühren bei 175-180 °C zugegeben. Es wird Kohlendioxid frei. Der Zulauf kann gegebenenfalls bis auf 10 h verlängert werden, je nach technischer Möglichkeit das Kohlendioxid abzuführen.
   4. Für die Nachreaktion wird die Temperatur bei 175 °C bis 180 °C für 1-2 Stunden gehalten, gegebenenfalls auch länger, bis kein Kohlendioxid mehr entsteht und die Reaktion abgeschlossen ist.
   5. Das Reaktionsgemisch wird auf 150 °C abgekühlt und es werden 3,47 kg Salicylsäure zugesetzt.
   6. Wenn das Produkt weiter abgekühlt ist (50 °C bis 60 °C), kann es ohne Verwendung eines Filters in einen Hobbock abgelassen werden.
b) Herstellung von propoxylierten Bisphenol F (PBF)
   1. 632,50 kg Bisphenol F werden als Feststoff in einen Reaktor gegeben und bei Temperaturen zwischen 120 °C - 160 °C aufgeschmolzen.
   2. 1,58 kg Kaliumcarbonat werden anschließend unter Rühren bei 130 °C zugegeben und die Reaktionsmischung weiter auf 175 °C - 180 °C aufgeheizt.
   3. Danach werden 645,80 kg Propylencarbonat in 5 h unter Rühren bei 175-180 °C zugegeben. Es wird Kohlendioxid frei. Der Zulauf kann gegebenenfalls bis auf 10 h verlängert werden, je nach technischer Möglichkeit das Kohlendioxid abzuführen.
   4. Für die Nachreaktion wird die Temperatur bei 175 °C bis 180 °C für 1-3 Stunden gehalten, gegebenenfalls auch länger, bis kein Kohlendioxid mehr entsteht und die Reaktion abgeschlossen ist.
   5. Das Reaktionsgemisch wird auf 150 °C abgekühlt und es werden 3,16 kg Salicylsäure zugesetzt.
   6. Um den Gehalt an freiem Propylencarbonat von 0,3 % bis auf < 0,1 % zu reduzieren kann gegebenenfalls unter Vakuum dieses abdestilliert werden.
   7. Wenn das Produkt weiter abgekühlt ist (50 °C bis 60 °C) kann es ohne Verwendung eines Filters in einen Hobbock abgelassen werden.
c) Herstellen des alkoxylierten Resorcins (Molverhältnis Resorcin : Propylencarbonat: Ethylencarbonat = 1 : 1,0 : 1,0)
   1. 516,7 kg Resorcin werden als Feststoff in einen Reaktor gegeben und aufgeschmolzen (Fp: 111 °C).
   2. 1,31 kg Kaliumcarbonat werden anschließend unter Rühren bei 130 °C zugegeben und die Reaktionsmischung weiter auf 175 °C - 180 °C aufgeheizt.
   3. Danach werden 479,1 kg Propylencarbonat in 2,5 h unter Rühren bei 175-180 °C zugegeben. Es wird Kohlendioxid frei. Der Zulauf kann gegebenenfalls auf bis zu 5 h verlängert werden, je nach technischer Möglichkeit das Kohlendioxid abzuführen.
   4. Anschließend werden 413,4 kg Ethylencarbonat in 2,5 h unter Rühren bei 175-180 °C zugegeben. Es wird wiederum Kohlendioxid frei. Der Zulauf kann gegebenenfalls bis zu 5 h verlängert werden, je nach technischer Möglichkeit das Kohlendioxid abzuführen.
   5. Für die Nachreaktion wird die Temperatur bei 175 °C bis 180 °C für 2-6 Stunden gehalten, gegebenenfalls auch länger, bis kein Kohlendioxid mehr entsteht und die Reaktion abgeschlossen ist.
   6. Das Reaktionsgemisch wird im Vakuum bei 175 - 180 °C kurz destilliert.
   7. Das Reaktionsgemisch wird auf 140 °C abgekühlt und es werden 2,58 kg Salicylsäure zugesetzt.
d) Herstellen des propoxylierten Resorcins (Molverhältnis Resorcin : Propylencarbonat = 1 : 2,0)
   Die Herstellung des propoxylierten Resorcins erfolgte wie unter c) beschrieben jedoch wurden 485,1 kg Resorcin unter Verwendung von 1,21 kg Kaliumcarbonat mit 899, 6 kg Propylencarbonat umgesetzt, so dass auf Schritt 4. verzichtet wurde.

Das unter a) und b) hergestellte ethoxylierte bzw. propoxylierte Bisphenol F wurde den in den Tabellen 1 und 2 angegebenen Lösungsmitteln zugesetzt. Dabei wurde das Gewichtsverhältnis von ethoxyliertem Bisphenol F : propoxyliertem Bisphenol F 50:50 gewählt.

Das Gewichtsverhältnis dieser binären Mischung zum angegebenen Lösungsmittel betrug 80 : 20 bzw. 50:50. Das ethoxylierte bzw. propoxylierte Bisphenol F wurde unter Rühren in das Lösungsmittel eingebracht und zur Lagerung in einen Klimaschrank gestellt. Nach der angegebenen Zeit (eine Woche - Tabelle 1; 7 Wochen - Tabelle 2) wurden die Proben entnommen und hinsichtlich ihrer Auskristallisation begutachtet. Aufgrund der Konsistenz der Proben war eine quantitative Analyse des auskristallisierten Produktes nicht möglich, so dass eine Bestimmung des Kristallisationsgrades mittels visuellen Vergleichs der Proben untereinander erfolgte. Dabei wurde die vollständige Auskristallisation 100 % und keine Auskristallisation 0 % gesetzt. Die Abstufung der Auskristallisation wurde mehrfach visuell bestimmt.

**Tabelle 1 - Lagerung eine Woche bei 20 °C im Klimaschrank**

| Lösungsmittel | Verhältnis x : Lösungsmittel | x | | |
|---|---|---|---|---|
| | | Ethoxyliertes Bisphenol F (EBF) | Propoxyliertes Bisphenol F (PBF) | 50 EBF : 50 PBF Erfindung |
| | | Auskristallisation | | |
| Triethylphosphat (TEP) | 80:20 | 100 % | 100 % | 100 % |
| | 50:50 | 100 % | 100 % | 50 % |
| Diethylenglykol (DEG) | 80:20 | 100 % | 100 % | 50 % |
| | 50:50 | 100 % | 100 % | 0 % |
| Ethoxylierter Zucker (Su-EO) | 80:20 | 100 % | 100 % | 50 % |
| | 50:50 | 100 % | 100 % | 0 % |
| Propoxyliertes Resorcin (PRC) | 80:20 | 100 % | 100 % | 0 % |
| | 50:50 | 100 % | 0 % | 0 % |
| 1,4-Butandiol | 80:20 | 100 % | 100 % | 50 % |
| | 50:50 | 100 % | 10 % | 10 % |
| Ethoxyliertes Phenol | 80:20 | 100 % | 100 % | 75 % |
| | 50:50 | 100 % | 100 % | 0 % |
| Ethoxyliertes o-Kresol | 80:20 | 100 % | 100% | 25 % |
| | 50:50 | 100 % | 100 % | 0 % |

Einige der Proben wurden noch weitere Wochen im Klimaschrank bei 20 °C aufbewahrt.

**Tabelle 2 - Lagerung 7 Wochen bei 20 °C im Klimaschrank**

| Lösungsmittel | Verhältnis x : Lösungsmittel | x | | |
|---|---|---|---|---|
| | | Ethoxyliertes Bisphenol F (EBF) | Propoxyliertes Bisphenol F (PBF) | 50 EBF : 50 PBF Erfindung |
| | | Auskristallisation | | |
| Diethylenglykol (DEG) | 50:50 | 100 % | 100 % | 75 % |
| Ethoxylierter Zucker (Su-EO) | 50:50 | 100 % | 100 % | 10 % |
| Propoxyliertes Resorcin (PRC) | 50:50 | 100 % | 50 % | 0 % |
| Ethoxyliertes Phenol | 50:50 | 100 % | 100 % | 50 % |
| Ethoxyliertes o-Kresol | 50:50 | 100 % | 100 % | 10 % |

Da die Auskristallisation der binären Mischung überrascherweise auch nach längere Zeit nur in einem begrenzten Umfang eintritt, ist es ersichtlich, dass die erfindungsgemäße Zusammensetzung über einen längeren Zeitraum lagerstabil ist. Auch bleibt die Viskosität der erfindungsgemäßen Zusammensetzung aufgrund der geringen Kristallisationsneigung nahezu unverändert, so dass diese als Polyolkomponente verwendet, eine gute Kompatibilität mit dem Isocyanat aufweist. Durch den hohen aromatischen Anteil der binären Mischung und gegebenenfalls zusätzlich noch durch die Verwendung von propoxyliertem Resorcin, was den aromatischen Anteil weiter erhöht, konnte eine gute Flammbeständigkeit im Polyurethan-Endprodukt erzielt werden, wobei auch die Kompatibilität zum MDI bzw. zu den Treibmitteln weiter erhöht werden konnte. Auf den zusätzlichen Einsatz von halogenierten Flammschutzmitteln konnte demnach verzichtet werden.

## Patentansprüche

1. Zusammensetzung eine binäre Mischung aus ethoxyliertem Bisphenol F und propoxyliertem Bisphenol F in einem Gewichtsverhältnis von 20: 80 bis 80:20 enthaltend.

2. Zusammensetzung nach Anspruch 1, **dadurch gekennzeichnet, dass** das ethoxylierte Bisphenol F durch Umsetzung von Bishenol F und Ethylencarbonat erhalten ist.

3. Zusammensetzung nach Anspruch 1, **dadurch gekennzeichnet, dass** das propoxylierte Bisphenol F durch Umsetzung von Bishenol F und Propylencarbonat erhalten ist.

4. Zusammensetzung nach zumindest einem der vorhergehenden Ansprüche, **dadurch gekennzeichnet, dass** das molare Verhältnis Bisphenol F : Ethoxylierungsmittel mindestens 1: 2 beträgt und/oder das Verhältnis Bisphenol F : Propoxylierungsmittel mindestens 1 : 2 beträgt.

5. Zusammensetzung nach zumindest einem der vorhergehenden Ansprüche, **dadurch gekennzeichnet, dass** das Gewichtsverhältnis ethoxyliertes Bisphenol F zu propoxyliertes Bisphenol F 30:70 bis 70:30 beträgt.

6. Zusammensetzung nach zumindest einem der vorhergehenden Ansprüche, **dadurch gekennzeichnet, dass** sie als weitere Komponente ein alkoxyliertes Resorcin enthält.

7. Zusammensetzung nach Anspruch 6, **dadurch gekennzeichnet, dass** das alkoxylierte Resorcin durch Umsetzung von Resorcin mit einer Kombination aus Ethoxylierungsmittel und Propoxylierungsmittel hergestellt ist.

8. Zusammensetzung nach Anspruch 6, **dadurch gekennzeichnet, dass** die binäre Mischung zum alkoxylierten Resorcin im Gewichtsverhältnis 80:20 bis 20:80 vorliegt.

9. Verfahren zur Herstellung einer Zusammensetzung nach Anspruch 1 folgende Schritte enthaltend:
I)
a) Zugabe von Bisphenol F in einen Reaktor und bei Temperaturen zwischen 120 °C und 160 °C aufschmelzen
b) Zugabe eines alkalischen Mediums und temperieren bis auf 180 °C
c) Zugabe von Ethylencarbonat und Abführung des Kohlendioxides
d) gegebenenfalls Zugabe einer Säure zur Neutralisation
II)
e) Zugabe von Bisphenol F in einen Reaktor und bei Temperaturen zwischen 120 °C und 160 °C aufschmelzen
f) Zugabe eines alkalischen Mediums und temperieren bis auf 180 °C
g) Zugabe von Propylencarbonat und Abführung des Kohlendioxides
h) gegebenenfalls Zugabe einer Säure zur Neutralisation
III) Zusammenführen der unter (I) und (II) hergestellten Komponenten, bevorzugt in einem Lösungsmittel, zur Herstellung der binären Mischung.

10. Verwendung der Zusammensetzung nach zu zumindest einem der vorhergehenden Ansprüche 1 bis 8 zur Herstellung von Polyurethanen oder Polyisocyanuraten.

11. Verwendung nach Anspruch 10 zur Herstellung von Polyurethanen in Form von Präpolymeren, Schäumen, Isolationsmaterialien, Beschichtungen, Lacken, Elastomeren, Klebstoffen, Dichtmitteln und/oder Kompositwerkstoffen.
